(19) **Europäisches Patentamt**
**European Patent Office**
**Office européen des brevets**

(11) **EP 1 952 754 B1**

(12) **EUROPEAN PATENT SPECIFICATION**

(45) Date of publication and mention
of the grant of the patent:
**07.03.2012 Bulletin 2012/10**

(51) Int Cl.:
*A61B 1/05* (2006.01)        *A61B 5/07* (2006.01)
*H02J 17/00* (2006.01)        *A61N 1/378* (2006.01)

(21) Application number: **08150501.8**

(22) Date of filing: **22.01.2008**

(54) **Wireless power supply system, capsulated endoscope, and capsulated endoscopic system**

Drahtloses Stromversorgungssystem, gekapseltes Endoskop und gekapseltes endoskopisches System

Système d'alimentation électrique sans fil, endoscope capsulé, et système endoscopique capsulé

(84) Designated Contracting States:
**DE FR GB**

(30) Priority: **24.01.2007 JP 2007014170**

(43) Date of publication of application:
**06.08.2008 Bulletin 2008/32**

(73) Proprietor: **Olympus Corporation**
**Shibuya-ku**
**Tokyo (JP)**

(72) Inventors:
• **Yoshida, Naoki**
**Tokyo 151-0072 (JP)**

• **Sakai, Youhei**
**Tokyo 151-0072 (JP)**
• **Iwaisako, Hiroshi**
**Tokyo 151-0072 (JP)**

(74) Representative: **Winter, Brandl, Fürniss, Hübner, Röss, Kaiser, Polte - Partnerschaft**
**Alois-Steinecker-Strasse 22**
**85354 Freising (DE)**

(56) References cited:
**WO-A-01/52351**          **JP-A- 2006 149 687**
**US-A- 5 741 316**         **US-A1- 2002 165 592**
**US-A1- 2005 216 231**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to a capsulated endoscope equipped with the wireless power supply system according to claim 1, and a capsulated endoscopic system in accordance with claim 4.

2. Description of the Related Art

Generally, an insertion portion of an endoscope in an endoscopic system, which is inserted into a body cavity has its diameter and length limited. Accordingly, the range which allows an operator to perform the observation, inspection and the like is restricted.

Recently, an ultra-compact endoscope with a built-in image pickup device equipped with a shooting optical system inside a tablet capsule-shaped outer case, that is, a capsulated endoscope has been developed for coping with the aforementioned limitation.

The capsulated endoscope is taken into the body cavity of a subject through a certain process such as swallowing. The capsulated endoscope swallowed by the subject is moved in the body cavity by a peristaltic motion of intestines, for example such that an image of an affected site of an internal organ in the body cavity is shot by the image pickup device, and the endoscopic image is stored in a memory, or wirelessly transmitted to a receiver unit outside the subject body.

The capsulated endoscope allows the endoscopic image signal which has been picked up to be received outside the body through the wireless communication such that the operator is capable of easily observing the endoscopic image. The capsulated endoscope allows the operator to easily observe the endoscopic image by taking the memory of the capsulated endoscope ejected from the body to obtain the picked up endoscopic image stored in the memory. In this way, the use of the capsulated endoscope allows the operator to easily observe and inspect and the like inside the body cavity of the subject.

The use of the capsulated endoscope for inspecting inside the body cavity makes it possible to relatively easily perform observation and inspection of the internal organ such as the small intestine, which have been difficult for the generally employed endoscope equipped with the thin and long insertion portion.

Generally, as the source of power for the aforementioned capsulated endoscope, a battery contained therein has been employed. Recently, the system for eliminating interruption of the operation of the capsulated endoscope owing to run down of the battery has been considered. The wireless power feeding process is one of the aforementioned systems in consideration. In case of the wireless power feeding, the electric power is fed from the power feeding system outside the body. The electric power is supplied from the outlet to the power feeding system so as to allow the capsulated endoscope to be used permanently.

As disclosed in Japanese Unexamined Patent Application Publication No. 2001-224551, the wireless power feeding has been generally performed as described below.

The power feeding system outside the body is generally structured to allow the control circuit and the drive circuit therein to drive a transmission antenna to generate an AC magnetic field. The generated AC magnetic field passes through the subject body to reach the capsulated endoscope which has been taken into the body.

Meanwhile, the capsulated endoscope contains a receiver antenna and a receiver circuit formed of a rectifying circuit and a smoothing circuit. The magnetic flux generated in the AC magnetic field flows to the contained receiver antenna to generate electricity at both ends of the receiver antenna, which will be rectified and smoothed to serve as the source of power.

Additionally, Japanese Unexamined Patent Application JP 2006-149687A discloses a capsule type endoscope comprising a wireless power supply system including a power feeding system equipped with a transmission antenna for wirelessly transmitting an electric power from a power source and a receiver antenna having a receiver coil wound around an outer periphery of a substantially bar-like core for receiving the transmitted electric power, wherein the core of the receiver antenna has at least one end area tilting at a predetermined angle with respect to a linear area.

Japanese Unexamined Patent Application Publication No. 2001-224551 discloses the capsulated endoscope provided with illumination means for illuminating inside the living body, image pickup means for picking up the image of the portion illuminated by the illumination means, a transmission antenna for wirelessly transmitting the image signal obtained by the image pickup means, and a receiver coil for receiving the electric power transmitted from the outside the body.

The generally employed capsulated endoscope is structured to allow the signal processing section to convert the electric charge photoelectrically converted by the image pickup means into an image signal, and to modulate/amplify the signal through the modulation/transmission amplifier into the transmission signal so as to be emitted outside the body from the transmission antenna as the electric wave. The capsulated endoscope includes the receiver coil.

The receiver coil is formed as a cylindrical shape to surround the inner wall of the capsule. The AC magnetic field generated outside passes through the body to reach the capsulated endoscope which has been taken therein. The magnetic flux generated in the AC magnetic field acts on the receiver coil to generate the electricity at both ends. The thus generated electricity is rectified and smoothed so as to serve as the source of power for the capsulated endoscope.

Conventionally, the electricity is wirelessly supplied from the outside the body to feed electric power to the capsulated endoscope as needed.

The generally employed capsulated endoscope moves in the complex internal organs by changing its orientation. Accordingly, there may be the case where the direction of the winding axis of the receiver coil which forms the receiver antenna in the capsulated endoscope disaccords with that of the magnetic flux generated by the transmission antenna.

Assuming that the angle formed by interlinking of the receiver antenna with the magnetic flux is defined as angle $\theta$, the electricity received by the capsulated endoscope becomes maximum when $\theta = 0°$. The electricity is reduced as $\theta$ becomes large. In order to supply sufficient electric power to the capsulated endoscope, the magnetic flux larger than that in the case where $\theta = 0°$, that is, high feeding power is required. The diameter of the receiver antenna should be made large for improving the power receiving efficiency. If the diameter of the receiver antenna is made large, the size of the capsulated endoscope itself is increased. This makes it difficult for the subject to swallow the capsulated endoscope, or the swallowed capsulated endoscope may get stuck in the internal organ.

Accordingly, it is an object of the present invention to allow the conventional power feeding to generate sufficient drive power, save energy of the wireless power supply system, and reduce the size of the capsulated endoscope by improving the power receiving efficiency of the wireless power supply system applicable to the capsulated endoscope without increasing the size of the capsulated endoscope.

The above object is achieved by a capsulated endoscope according to claim 1, and a capsulated endoscopic system in accordance with claim 4.

SUMMARY OF THE INVENTION

Underlying the present invention, a wireless power supply system includes a power feeding system equipped with a transmission antenna for wirelessly transmitting an electric power from a power source, and a receiver antenna having a receiver coil wound around an outer periphery of a substantially bar-like core for receiving the transmitted electric power. The core of the receiver antenna has at least one end area tilting at a predetermined angle with respect to a linear area.

In the first aspect of the present invention, a capsulated endoscope includes a receiver antenna having a receiver coil wound around an outer periphery of a substantially bar-like core for receiving an electric power wirelessly. The core of the receiver antenna has at least one end area tilting at a predetermined angle with respect to a linear area.

In the second aspect of the present invention, a capsulated endoscopic system includes a power feeding system equipped with a transmission antenna for wirelessly transmitting an electric power from a power source, and a capsulated endoscope which contains a receiver antenna having a receiver coil wound around an outer periphery of a substantially bar-like core for receiving the transmitted electric power. The core of the receiver antenna has at least one end area tilting at a predetermined angle with respect to a linear area.

The above and other objects, features and advantages of the invention will become more clearly understood from the following description referring to the accompanying drawings.

BRIEF DESCRIPTION OF THE DRAWINGS

Fig. 1 is a view showing a structure of a capsulated endoscopic system according to a first embodiment, and a state where a capsulated endoscope has been taken into a body cavity of a subject body.

Fig. 2 is a cross-sectional view of the capsulated endoscope equipped with a receiver antenna.

Fig. 3 is a cross-sectional view taken along line III-III shown in Fig. 2 as a front view of the capsulated endoscope in a cylindrical direction.

Fig. 4 is a block diagram showing the capsulated endoscope and the power feeding system.

Fig. 5 is a view showing the structure of the receiver antenna equipped with a straight core.

Fig. 6 is a view showing the structure of the receiver antenna with the core having both ends folded at 30°, respectively.

Fig. 7 is a view showing the structure of the receiver antenna with the core having both ends folded at 45°, respectively.

Fig. 8 is a view showing the structure of the receiver antenna with the core having both ends folded at 30°, which is different from the one shown in Fig. 6.

Fig. 9 is a view showing the state where the receiver antenna is located in the magnetic field.

Fig. 10 is a graph showing each power receiving state of the straight core and the folded core.

Fig. 11 is a cross-sectional view seen from a front of the capsulated endoscope according to a second embodiment.

Fig. 12 is a partially cross-sectional view of the capsulated endoscope taken along line XII-XII shown in Fig. 11.

Fig. 13 is a first view showing an example of an electric coupling between two receiver antennas.

Fig. 14 is a second view showing another example of the electric coupling between the two receiver antennas.

Fig. 15 is a third view showing another example of the electric coupling between the two receiver antennas.

Fig. 16 is a fourth view showing another example of the electric coupling between the two receiver antennas.

Fig. 17 is a view showing the state of the magnetic flux collected by the core.

Fig. 18 is a view showing the state of the magnetic flux collected by the core which is the same as the one shown in Fig. 17.

Fig. 19 is a view showing the state of the magnetic flux collected by the core with the volume equal to the sum of volumes of the cores shown in Figs. 17 and 18.

Fig. 20 is a cross-sectional view seen from the front of the capsulated endoscope according to a third embodiment.

Fig. 21 is a cross-sectional view of the capsulated endoscope taken along line XXI-XXI shown in Fig. 20.

Fig. 22 is a cross-sectional view seen from the front of the capsulated endoscope indicating an arrangement of four receiver antennas as a first modified example.

Fig. 23 is a cross-sectional view seen from the front of the capsulated endoscope indicating the arrangement of three receiver antennas as a second modified example.

Fig. 24 is a cross-sectional view of the capsulated endoscope as a third modified example.

## DETAILED DESCRIPTION OF THE PREFERRED EMBODIMENTS

Embodiments of the present invention will be described referring to the drawings. The wireless power supply system according to the embodiments as below will be described with respect to the capsulated endoscope.

First Embodiment

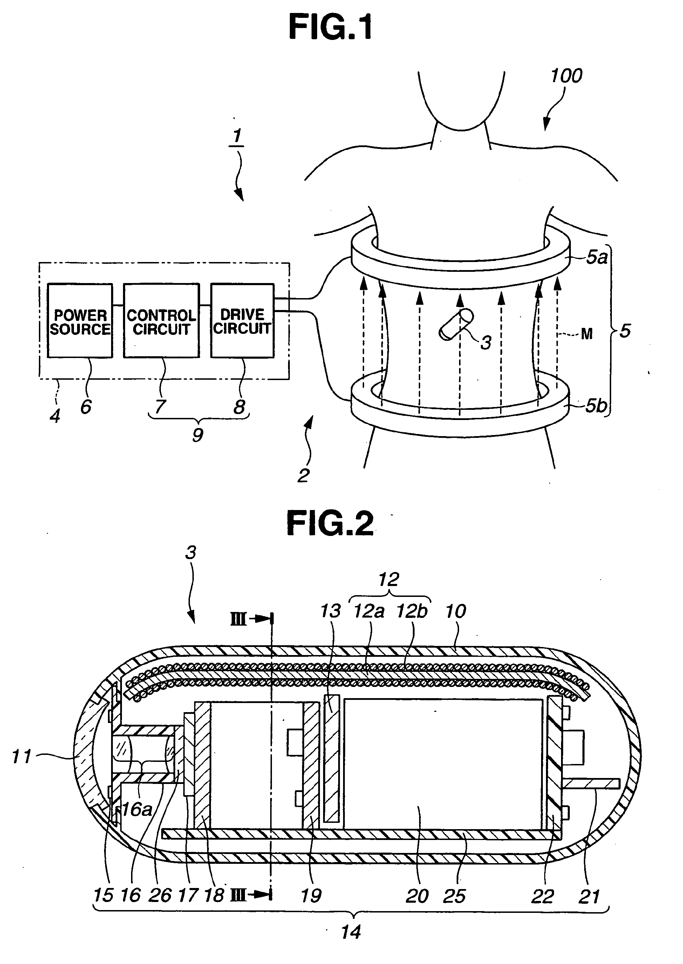

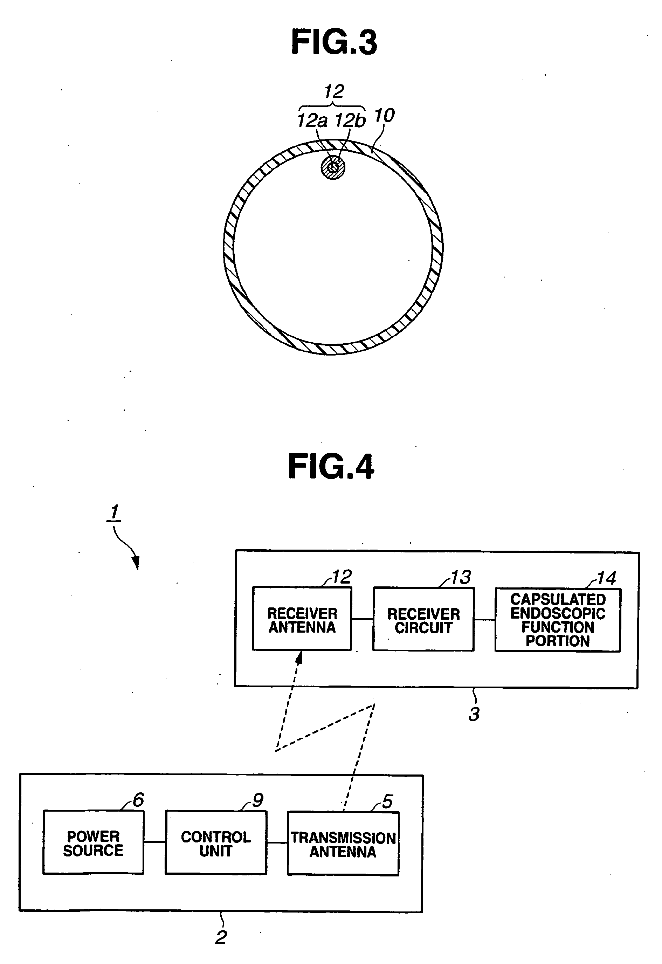

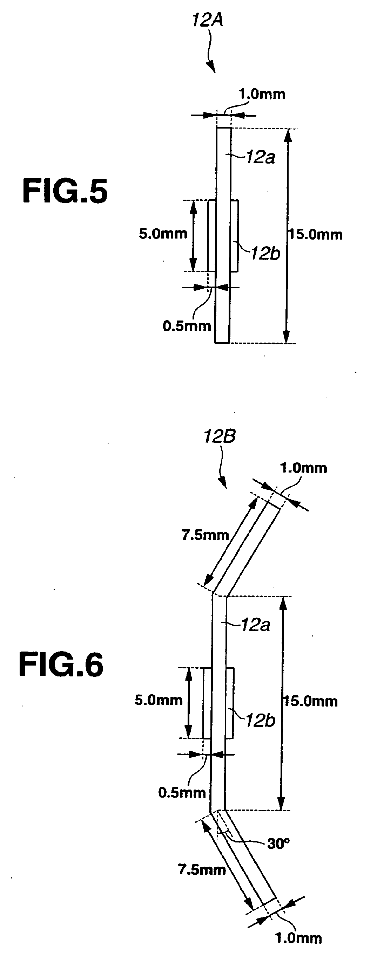

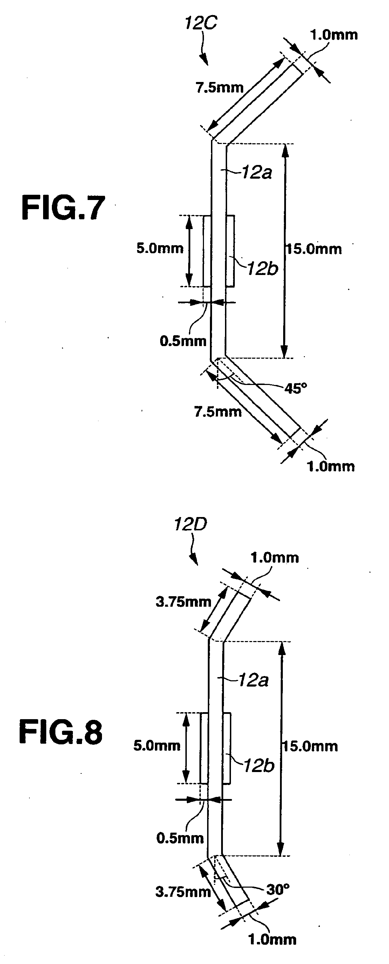

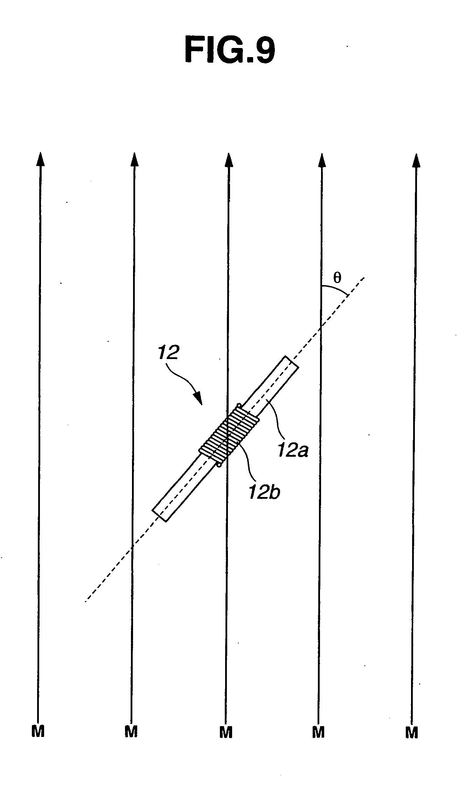

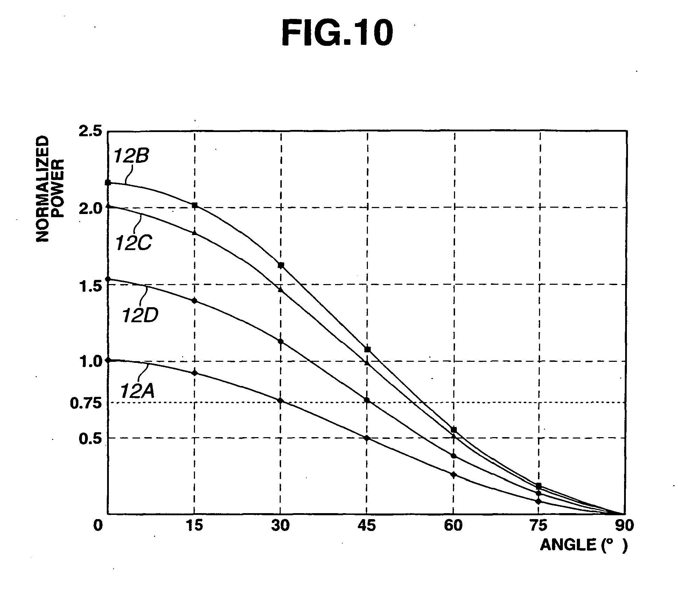

A first embodiment of the present invention will be described referring to Figs. 1 to 10. Fig. 1 is a view showing a structure of a capsulated endoscopic system, and a state where a capsulated endoscope has been taken into a body cavity of a subject body. Fig. 2 is a cross-sectional view of the capsulated endoscope equipped with a receiver antenna. Fig. 3 is a cross-sectional view taken along line III-III shown in Fig. 2 as a front view of the capsulated endoscope in a cylindrical direction. Fig. 4 is a block diagram showing the capsulated endoscope and the power feeding system. Fig. 5 is a view showing the structure of the receiver antenna equipped with a straight core. Fig. 6 is a view showing the structure of the receiver antenna with the core having both ends folded at 30°, respectively. Fig. 7 is a view showing the structure of the receiver antenna with the core having both ends folded at 45°, respectively. Fig. 8 is a view showing the structure of the receiver antenna with the core having both ends folded at 30°, which is different from the one shown in Fig. 6. Fig. 9 is a view showing the state where the receiver antenna is located in the magnetic field. Fig. 10 is a graph showing each power receiving state of the straight core and the folded core.

Referring to Fig. 1, a capsulated endoscopic system 1 according to the embodiment includes a power feeding system 2 and a capsulated endoscope 3 which form the wireless power supply system.

The power feeding system 2 formed of a main body 4 and a transmission antenna 5 is placed outside the subject body. The main body 4 contains a power source 6, a control circuit 7 and a drive circuit 8 which are electrically coupled with one another. In the embodiment, the control circuit 7 and the drive circuit 8 form a control unit 9.

The transmission antenna 5 is formed of two ring-like antenna portions 5a and 5b as Helmholtz coils which are electrically coupled with the drive circuit 8 of the main body 4. Those antenna portions 5a and 5b are put on a torso of a subject 100 so as to be apart from each other by a predetermined interval.

The above-structured power feeding system 2 applies alternate current generated by the power source 6 and the control circuit 7 to the transmission antenna 5. Upon reception of the alternate current, the transmission antenna 5 generates the AC magnetic field corresponding to the applied alternate current to generate a magnetic flux M directed from the antenna portion 5b to the antenna portion 5a in the body of the subject. Besides the Helmholtz coil, an arbitrary coil, for example, the solenoid coil or other type of coil may be employed as the transmission antenna 5.

A capsulated endoscope 3 with a capsule tablet-like outer appearance as shown in Fig. 1 is swallowed by the subject to be taken into the body cavity.

Referring to Fig. 2, the capsulated endoscope 3 are sealed by an outer case 10 of a substantially capsule type having both ends dome-like formed, and a transparent cover 11 set at one end of the outer case 10. A receiver antenna 12, a receiver circuit 13, and a capsulated endoscopic function portion 14 are placed inside the outer case 10.

The receiver antenna 12 includes a core 12a formed of a magnetic material, and a receiver coil 12b wound around the outer periphery of the core 12a.

The core 12a is formed of a high magnetic permeability magnetic material, for example, a metal alloy material, a ferrite material, and an amorphous magnetic material. As shown in Fig. 3, the core 12a is placed adjacent to the inner periphery of the outer case 10 to have a long bar-like shape. Referring to Fig. 2, the core 12a includes a linear area, and bent areas extending from both ends of the linear area along the dome-like bent portion of the inner wall of the outer case 10.

In the embodiment, the core 12a is formed to have both ends along the bent inner wall of the outer case 10 rather than linearly formed to have the length as long as possible. The present embodiment increases the total length of the core 12a for collecting the magnetic flux M without increasing the size of the capsulated endoscope 3.

The receiver circuit 13 includes a rectifying circuit such as a diode bridge for rectifying the alternate current and a smoothing circuit such as a capacitor functioning as an energy storage element and a ripple attenuation element in the capsulated endoscope for conversion into the electric power received by the receiver antenna 12. Explanation and illustration of those generally employed circuits will be omitted.

Illustration of a resonant capacitor connected to the receiver antenna 12 so as to be resonated with the frequency of the externally applied AC magnetic field will also be omitted.

The capsulated endoscopic function portion 14 includes an LED 15 as illumination means, a lens frame 16 provided with a lens group 16a as an image pickup optical system, an image pickup device drive unit 17 provided with an image pickup device 26 serving as an image sensor such as a CMOS and a CCD, a signal processing unit 18, a receiver circuit 19, an emergency battery 20, and a modulation transmission amplifier 22 provided with a transmission antenna 21 for transmitting the image which has been shot to the outside. The aforementioned elements in a predetermined arrangement are disposed on a rigid substrate or an FPC electric substrate 25.

The operation of the above-structured capsulated endoscopic system 1 according to the embodiment will be described.

The subject in the AC magnetic field as shown in Fig. 1 swallows the capsulated endoscope 3 so as to be taken into the body cavity and moved by peristaltic motions of the intestines, for example along the digestive tract.

The capsulated endoscope 3 receives the magnetic flux M externally generated by the AC magnetic field by the receiver antenna 12 while moving so as to be converted into the electric power. The resultant electric power enables all the electric functions of the capsulated endoscopic function portion 14 in the capsulated endoscope 3.

In other words, the electric power from the power source 6 of the power feeding system 2 generates the magnetic flux M in the AC magnetic field by the transmission antenna 5 via the control unit 9 as shown in Fig. 4. Then the capsulated endoscope 3 receives the magnetic flux M by the receiver antenna 12 such that the receiver circuit 13 feeds the electricity to the respective electric components of the capsulated endoscopic function portion 14.

In the aforementioned case, the core 12a is disposed to have both ends following along the inner wall of the outer case 10 and extending to the dome-like bent portions to the front and rear of the outer case 10, respectively. The influence of the demagnetizing field generated inside the core 12a is reduced such that more magnetic flux M is collected. This makes it possible to improve the power receiving capability.

The influence to the power receiving capability in accordance with the length of the core 12a will be described referring to Figs. 5 to 10. Four different types of receiver antennas 12A to 12D as shown in Figs. 5 to 8 are used for the verification.

The receiver antenna 12A shown in Fig. 5 includes a receiver coil 12b wound around a center of a cylindrical core 12a with a total length of 15.0 mm and a diameter of 1.0 mm by 50 times at the winding thickness of 5.0 mm.

The receiver antenna 12B having both ends folded as shown in Fig. 6 includes a receiver coil 12b wound around the center of the core 12a with a linear length of 15.0 mm and each length of the folded portions of 7.5 mm by 50 times at the winding thickness of 5.0 mm. Each end of the antenna is folded at an angle of 30° with respect to the linear area such that the areas at both ends tilt in the same direction.

The receiver antenna 12C having both ends folded as shown in Fig. 7 likewise the receiver antenna 12B in Fig. 6 includes a receiver coil 12b wound around the center of a core 12a with a linear length of 15.0 mm and each length of the folded portions of 7.5 mm by 50 times at the winding thickness of 5.0 mm. Each end of the antenna is folded at an angle of 45° with respect to the linear area such that the areas at both ends tilt in the same direction.

The receiver antenna 12D having both ends folded as shown in Fig. 8 includes a receiver coil 12b wound around the center of a core 12a with a linear length of 15.0 mm and each length of the folded portions of 3.75 mm by 50 times at the winding thickness of 5.0 mm. Each end of the antenna is folded at an angle of 30° with respect to the linear area such that the areas at both ends tilt in the same direction likewise the receiver antenna 12B shown in Fig. 6.

Each of the cores 12a used in the aforementioned four types of the receiver antennas 12A to 12D is formed of a ferrite material. The receiver coil 12b used in those antennas is also formed of the ferrite material.

Each of those receiver antennas 12A to 12D is disposed in the magnetic field of the magnetic flux M generated by the AC magnetic field under the same condition of the transmission antenna 5 as shown in Fig. 9, respectively. It is assumed that the value of the electric power received when the angle θ defined by the longitudinal axis of the receiver antenna 12A and the direction of the magnetic flux M is zero (θ=0), that is, those directions are in the parallel state is set to 1.0. The electric power required for electrically driving the capsulated endoscopic function portion 14 in the capsulated endoscope 3 is set to the value of 0.75 or more, for example.

Fig. 10 shows each change in the electric power received by the respective receiver antennas 12A to 12D when the angle θ defined by interlinking of the axis of the linear area with the magnetic flux M is gradually increased by every 15°.

Referring to Fig. 10, in the case where the receiver antenna 12A is used while keeping the intensity value of the magnetic flux M constant, the received electric power value is below 0.75 as the angle with respect to the magnetic flux M direction becomes 30° or more (θ=30°). In this case, the resultant electric power is insufficient for electrically driving the capsulated endoscopic function portion 14.

The electric power value received by the receiver antenna 12D exceeds 0.75 so long as the angle θ is up to 45° (θ=45°). The resultant electric power sufficient for electrically driving the capsulated endoscopic function portion 14 may be maintained.

In both cases of the receiver antennas 12A and 12B, the received electric power value exceeds 0.75 at the angle θ of at least 50°. Therefore, the resultant electric power sufficient for electrically driving the capsulated endoscopic function portion 14 may be maintained.

It is clarified that the influence of demagnetizing field is reduced by increasing the total length of the core 12a for collecting more magnetic flux M to suppress the influence to the change in the angle. Comparison between the receiver antennas 12B and 12C shows that the power receiving efficiency is further improved to a slight degree in the case of the obtuse angle defined by each end portion and the linear area of the core 12a.

Comparison between the receiver antennas 12B and 12D shows that the longer the length of the area from the folded portion to each end becomes, the more power receiving efficiency is achieved in the case where both ends of the core 12a are folded at the same angle (30°) with respect to the linear area.

The receiver antenna 12 according to the embodiment is allowed to collect more magnetic flux M under less influence of the internally generated demagnetizing field by increasing the total length of the core 12a through extension up to the dome-like bent portion in the limited inner space of the capsulated endoscope 3. The thus structured receiver antenna 12 is capable of receiving sufficient electric power without increasing the electric power fed from the power feeding system 2 unnecessarily.

In the case where the receiver antenna 12 fails to receive sufficient electric power for electrically driving the capsulated endoscopic function portion 14, the capsulated endoscope 3 according to the embodiment allows the emergency battery 20 to feed the auxiliary electric power.

Second Embodiment

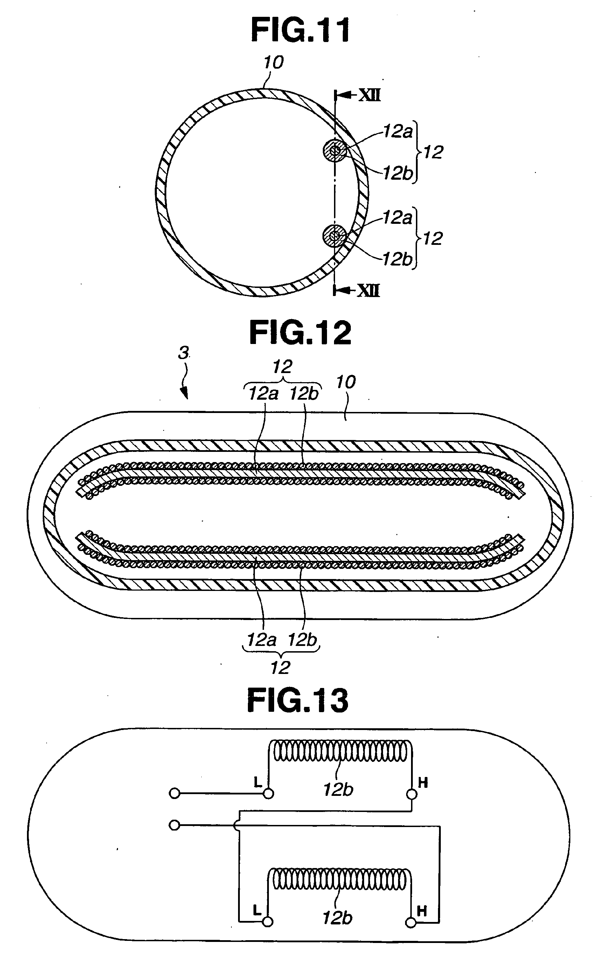

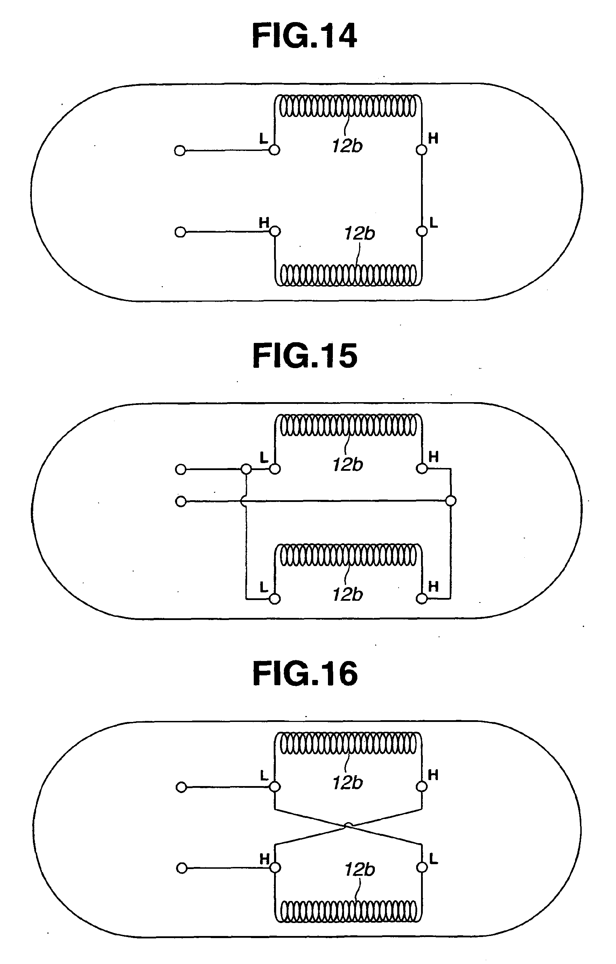

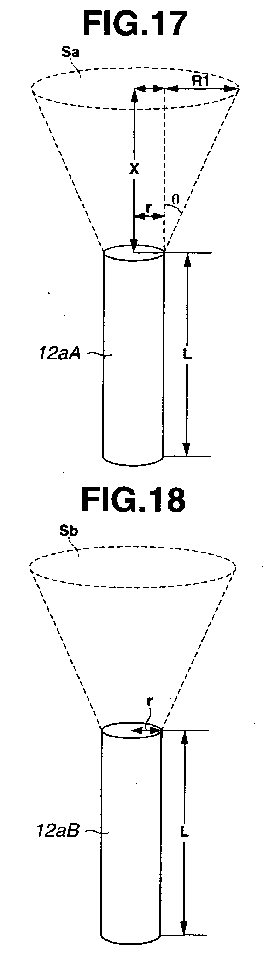

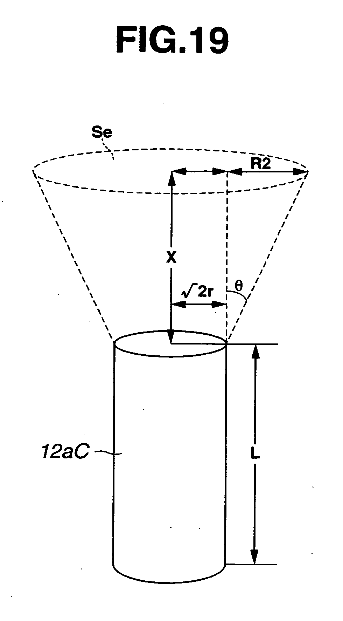

A second embodiment according to the present invention will be described referring to Figs. 11 to 19. Fig. 11 is a cross-sectional view seen from a front of the capsulated endoscope. Fig. 12 is a partially cross-sectional view of the capsulated endoscope taken along line XII-XII shown in Fig. 11. Fig. 13 is a first view showing an example of an electric coupling between two receiver antennas. Fig. 14 is a second view showing another example of the electric coupling between the two receiver antennas. Fig. 15 is a third view showing another example of the electric coupling between the two receiver antennas. Fig. 16 is a fourth view showing another example of the electric coupling between the two receiver antennas. Fig. 17 is a view showing the state of the magnetic flux collected by the core. Fig. 18 is a view showing the state of the magnetic flux collected by the core which is the same as the one shown in Fig. 17. Fig. 19 is a view showing the state of the magnetic flux collected by the core with the volume equal to the sum of volumes of the cores shown in Figs. 17 and 18.

In the following description, the same components as those of the capsulated endoscopic system 1 according to the first embodiment will be designated with the same reference numerals, and explanations thereof, thus will be omitted. Other components such as the known light source, the image pickup optical system, the image pickup device, and the electronically controlled unit contained in the capsulated endoscope are not illustrated for simplifying the drawing.

Referring to Figs. 11 and 12, two receiver antennas 12 each including the core 12a formed of the magnetic material around which the receiver coil 12b is wound are disposed inside the outer case 10 of the capsulated endoscope 3. The core 12a has both ends configured along the bent inner wall of the outer case 10 likewise the first embodiment. The receiver coils 12b of the receiver antennas 12 are electrically connected in series or in parallel.

The above-structured capsulated endoscope 3 according to the embodiment receives the magnetic flux M generated in the externally generated AC magnetic field by two receiver antennas 12 so as to be converted into the electric power while moving inside the subject body likewise the first embodiment. The resultant electric power enables all the electric functions within the capsule.

Referring to Fig. 12, the two receiver antennas 12 are disposed adjacent with each other inside the outer case 10 along the inner wall thereof. Likewise the first embodiment, the cores 12a extend to the dome-like bent portions to the front and rear of the outer case 10. The aforementioned arrangement increases the total length of the core 12a for collecting the magnetic flux M.

Likewise the first embodiment, the increase in the total length of the core makes it possible to collect more magnetic flux M by suppressing the influence of the demagnetizing field. The effective magnetic fluxes M interlinked with a plurality of receiver antennas are further enlarged compared with the first embodiment.

The process for electrically coupling the receiver coils 12b of those two receiver antennas 12 in the capsulated endoscope either in series or in parallel will be described referring to Figs. 13 to 16. In the following description, the illustration of the cores 12a will be omitted for simplifying the drawing.

The process for connecting the receiver coils in series will be described referring to Figs. 13 and 14.

Referring to Fig. 13, each of two receiver coils 12b is wound in the same direction. In this case, hetero poles of the receiver coils 12b are connected in series to form a series circuit through wiring such that the electric power generated at both ends of the receiver coils may be combined without dissipating the power.

Referring to Fig. 14, each of two receiver coils 12b is wound in the different direction. In this case, hetero poles of the receiver coils 12b are connected in series to form a series circuit through wiring such that the electric power generated at both ends of the receiver coils may be combined without dissipating the power.

The process for connecting the receiver coils in parallel will be described referring to Figs. 15 and 16.

Referring to Fig. 15, each of two receiver coils 12b is wound in the same direction. In this case, homo poles of the receiver coils 12b are connected in parallel to form a parallel circuit through wiring such that the electric power generated at both ends of the receiver coils may be combined without dissipating the power.

Referring to Fig. 16, each of two receiver coils 12b is wound in the different direction. In this case, homo poles of the receiver coils 12b are connected in parallel to form a parallel circuit through wiring such that the electric power generated at both ends of the receiver coils may be combined without dissipating the power.

The selection between the series connection and the parallel connection of the plural receiver antennas 12 is made based on the determination as to which is more advantageous that may change depending on the load condition. Accordingly, the series connection or the parallel connection may be selected in accordance with the load conditions.

The difference between the arrangement of the plural receiver antennas 12 and the arrangement of a single receiver antenna with the volume same as those of the plural receiver antennas 12 will be described referring to Figs. 17 to 19. The explanation is made in reference to the use of the linear core 12a for simplifying the drawing. However, the folded core 12a is also available.

As only core 12a of the receiver antenna 12 functions in collecting the magnetic flux M, Figs. 17 to 19 show the core 12a only.

Three cores, that is, a cylindrical core 12a with the radius of r and the length of L as shown in Fig. 17, a core 12aB shown in Fig. 18 with the same shape and dimension as those of the core 12aA in Fig. 17, and a cylindrical core 12aC with the radius of √2r and the length of L as shown in Fig. 19 are prepared. In other words, the sum of the volumes of the cores 12aA and 12aB is equivalent to that of the core 12aC.

Assuming that each area which allows collection of the magnetic flux M upon its emission to the respective cores 12aA to 12aC from a position apart from one end surface by the same distance X is set to Sa, Sb and Se, those areas may be expressed by the following formulae:

$$R1 = R2 = X\tan\theta \qquad \text{(Formula 1)}$$

$$Sa=Sb=\pi(r+X\tan\theta)2 \qquad \text{(Formula 2)}$$

$$Se=\pi(\sqrt{2}r + X\tan\theta)2 \qquad \text{(Formula 3)}$$

It is assumed that Xtanθ=R for simplifying the formula,

$$Sa=Sb=\pi(r+R)2 \qquad \text{(Formula 4)}$$

$$Sa+Sb=2\pi(r+R)2 \qquad\qquad \text{(Formula 5)}$$

$$Se=\pi(\sqrt{2}r +R)2 \qquad\qquad \text{(Formula 6)}$$

$$\text{(Formula 5) - (Formula 6)} = \pi(4-2\sqrt{2})rR > 0 \qquad \text{(Formula 7)}$$

In the formula 7, the area Sa + Sb is larger than the area Se. In the case where the receiver antenna 12 with the same volume is disposed in the limited space, it is better to dispose the plural receiver antennas 12 than to increase the diameter of the core 12a of the receiver antenna 12 for allowing more magnetic flux M to be collected under the limiting condition of the same volume.

If the plural receiver antennas 12 are prepared to be disposed in the limited space of the capsulated endoscope 3 by bending both end portions of the respective cores 12a to increase each total length of the plural cores 12a, the influence of the demagnetizing field is suppressed to allow more magnetic flux M to be collected.

As described above, in addition to the effect derived from the first embodiment, the embodiment allows reception of sufficient electric power without unnecessarily increasing the electric power fed from the power feeding system 2.

In the embodiment, the use of two receiver antennas 12 is described as the example. However, an arbitrary number of the receiver antennas of more than two may be set.

Third Embodiment

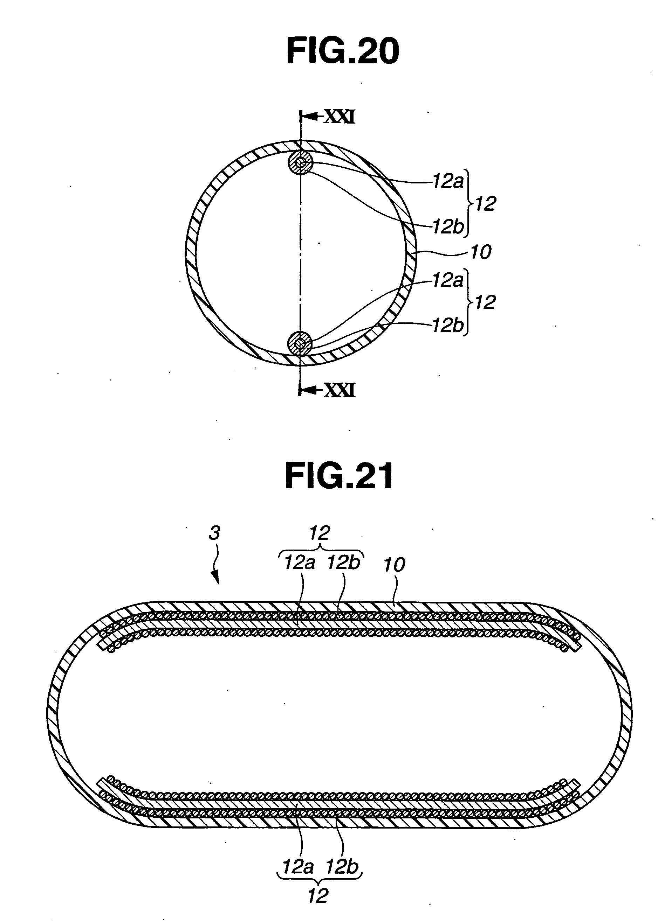

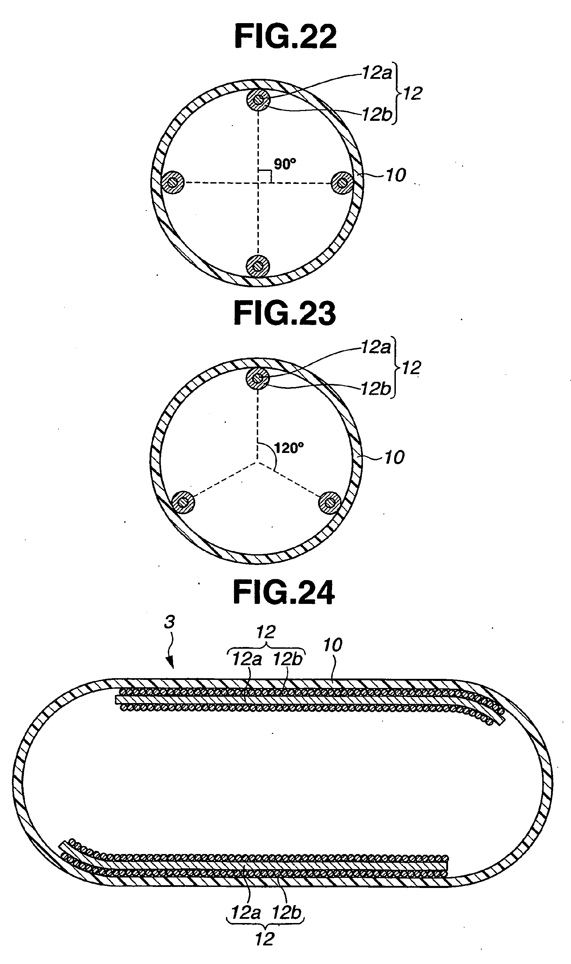

A third embodiment of the present invention will be described referring to Figs. 20 to 24. Fig. 20 is a cross-sectional view seen from the front of the capsulated endoscope. Fig. 21 is a cross-sectional view of the capsulated endoscope taken along line XXI-XXI shown in Fig. 20. Fig. 22 is a cross-sectional view seen from the front of the capsulated endoscope indicating an arrangement of four receiver antennas as a modified example. Fig. 23 is a cross-sectional view seen from the front of the capsulated endoscope indicating the arrangement of three receiver antennas as a modified example. Fig. 24 is a cross-sectional view of the capsulated endoscope as a modified example.

In the following description, the same components as those of the capsulated endoscopic system 1 according to the first and the second embodiments will be designated as the same reference numerals, and explanations thereof, thus will be omitted. The other components including the known light source, the image pickup optical system, the image pickup device, the electronically controlled unit and the like contained in the capsulated endoscope are not illustrated for simplifying the drawing.

The structure of the capsulated endoscope 3 of the embodiment is substantially the same as that of the capsulated endoscope 3 of the second embodiment except the positions where the plural receiver antennas 12 are arranged.

Referring to Figs. 20 and 21, two receiver antennas 12 are arranged at the positions so as to be the furthest apart from each other, in other words, diagonally arranged inside the outer case 10 along the inner wall thereof likewise the embodiments as described above. The aforementioned arrangement suppresses the influence owing to mutual induction of the respective receiver coils 12b, and increases each total length of the respective cores 12a for collecting the magnetic flux M.

The increase in each total length of the respective cores 12a suppresses the influence of the demagnetizing field, thus allowing more magnetic flux M to be collected. The plural cores 12a are arranged to enlarge the effective magnetic flux M interlinked therewith.

As the receiver antennas 12 are arranged the furthest apart from each other in the limited space of the capsulated endoscope 3, the influence of the mutual induction may be suppressed. Each total length of the respective cores 12a is increased to suppress the influence of the demagnetizing field to allow more magnetic flux M to be collected. The embodiment is capable of receiving sufficient electric power without increasing the electric power fed from the power feeding system 2 unnecessarily.

In the embodiment, the use of two receiver antennas 12 is described as the example. However, more than two receiver antennas may be used. For example, four receiver antennas 12 may be arranged each at the angular interval of 90° with respect to the center of the outer case 10 as shown in Fig. 21 so as to be the furthest apart from one another.

Alternatively, three receiver antennas 12 may also be arranged at the angular interval of 120° with respect to the center of the outer case 10 as shown in Fig. 22 so as to be the furthest apart from one another.

That is, n units of the receiver antennas 12 may be arranged at the angular interval of 360/n° with respect to

EP 1 952 754 B1

the center of the outer case 10. The n units of the receiver antennas 12 are arranged at the angular interval of 360/n° on the inner periphery of the outer case 10 with the circular cross section so as to suppress the influence of the mutual induction. If the cross section of the outer case 10 has other form such as the polygonal shape rather than the circular shape, the respective receiver antennas 12 are arranged so as to be the furthest apart from one another.

Referring to Fig. 13, each core 12a of the respective receiver antennas 12 may have only one end portion bent. The receiver antennas 12 are arranged such that one of the cores 12a extends to the dome-like bent portion to the front of the outer case 10, and the other of the cores 12a extends to the dome-like bent portion to the rear of the outer case 10 in the alternate arrangement state.

The aforementioned arrangement prevents each bent end area from being brought to be adjacent with each another, thus further suppressing the influence owing to the mutual induction of the respective receiver coils 12b.

In the present invention, the power receiving efficiency of the wireless power supply system applicable to the capsulated endoscope is improved without increasing the size of the capsulated endoscope itself to allow the general feeding electric power to generate the drive power, to save energy of the wireless power supply system, and reduce the size of the capsulated endoscope.

It is to be understood that the present invention formed as the respective embodiments may be modified into various forms at the stage of implementing the invention without departing from the scope of the invention. As the aforementioned embodiments contain the invention at various stages, various inventions may be extracted through the arbitrary combination of the disclosed plural elements.

For example, if the problem may be solved by the invention to provide the effect as described above even when a certain number of the elements are eliminated from all the elements introduced in the respective embodiments, the structure having the elements eliminated may be extracted as the invention.

**Claims**

1. A capsulated endoscope (3) comprising:

   a receiver antenna (12) having a receiver coil (12b) wound around an outer periphery of a substantially bar-like core (12a) for receiving an electric power wirelessly, wherein the core (12a) of the receiver antenna (12) has at least one end area tilting at a predetermined angle with respect to a linear area,
   **characterized in that**
   the at least one end area of the core (12a) is bent along a configuration of a case (10) which contains the receiver antenna (12).

2. The capsulated endoscope according to Claim 1, wherein:

   a plurality of the receiver antennas (12) are provided; and
   the receiver coils (12b) of the plurality of the receiver antennas (12) are connected in series or in parallel.

3. The capsulated endoscope according to Claim 2, wherein the plurality of the receiver antennas (12) are arranged the furthest apart from one another in the case (10).

4. A capsulated endoscopic system comprising:

   a power feeding system (2) equipped with a transmission antenna (5, 5a, 5b) for wirelessly transmitting an electric power from a power source (6); and
   a capsulated endoscope (3) which contains a receiver antenna (12) having a receiver coil (12b) wound around an outer periphery of a substantially bar-like core (12a) for receiving the transmitted electric power, wherein the core (12a) of the receiver antenna (12) has at least one end area tilting at a predetermined angle with respect to a linear area,
   **characterized in that**
   the at least one end area of the core (12a) is bent along a configuration of a case (10) which contains the receiver antenna (12).

5. The capsulated endoscopic system according to Claim 4, wherein:

   a plurality of the receiver antennas (12) are provided; and
   the receiver coils (12b) of the plurality of the receiver antennas (12) are connected in series or in parallel.

**6.** The capsulated endoscopic system according to Claim 5, wherein the plurality of the receiver antennas (12) are arranged the furthest apart from one another in the case (10).

**Patentansprüche**

**1.** Gekapseltes Endoskop (3) mit:

einer Empfangsantenne (12), die eine Empfangsspule (12b) aufweist, welche um eine äußere Peripherie eines im Wesentlichen stangenartigen Kerns (12a) gewunden ist, um drahtlos elektrische Leistung zu empfangen, wobei der Kern (12a) der Empfangsantenne (12) mindestens einen Bereich aufweist, der gegenüber einem linearen Bereich um einen vorab bestimmten Winkel gekippt ist,
**dadurch gekennzeichnet, dass**
der mindestens eine Endbereich des Kerns (12a) entlang eines Aufbaus eines Gehäuses (10) gebogen ist, das die Empfangsantenne (12) enthält.

**2.** Gekapseltes Endoskop (3) nach Anspruch 1, wobei:

eine Vielzahl von Empfangsantennen (12) vorhanden ist; und
die Empfangsspulen (12b) der Vielzahl der Empfangsantennen (12) in Reihe oder parallel verbunden sind.

**3.** Gekapseltes Endoskop nach Anspruch 2, wobei die Vielzahl von Empfangsantennen (12) so weit wie möglich voneinander beabstandet in dem Gehäuse (10) angeordnet sind.

**4.** Gekapseltes Endoskopsystem mit:

einer Leistungszuführungseinheit (2), die mit einer Übertragungsantenne (5, 5a, 5b) zum drahtlosen Übertragen einer elektrischen Leistung von einer Stromquelle (6) ausgestattet ist; und
einem gekapselten Endoskop (3), das eine Empfangsantenne (12) enthält, die eine Empfangsspule (12b) aufweist, welche um eine äußere Peripherie eines im Wesentlichen stangenartigen Kerns (12a) gewunden ist, um die übertragene elektrische Leistung zu empfangen, wobei der Kern (12a) der Empfangsantenne (12) mindestens einen Bereich aufweist, der gegenüber einem linearen Bereich um einen vorab bestimmten Winkel gekippt ist,
**dadurch gekennzeichnet, dass**
der mindestens eine Endbereich des Kerns (12a) entlang eines Aufbaus eines Gehäuses (10) gebogen ist, das die Empfangsantenne (12) enthält.

**5.** Gekapseltes Endoskopsystem nach Anspruch 4, wobei
eine Vielzahl von Empfangsantennen (12) vorhanden ist; und
die Empfangsspulen (12b) der Vielzahl der Empfangsantennen (12) in Reihe oder parallel verbunden sind.

**6.** Gekapseltes Endoskopsystem nach Anspruch 5, wobei die Vielzahl von Empfangsantennen (12) so weit wie möglich voneinander beabstandet in dem Gehäuse (10) angeordnet sind.

**Revendications**

**1.** Endoscope capsulé (3) comprenant :

une antenne réceptrice (12) ayant une bobine réceptrice (12b) enroulée autour d'une périphérie externe d'un noyau sensiblement en forme de barre (12a) pour recevoir une énergie électrique sans fil, dans lequel le noyau (12a) de l'antenne réceptrice (12) présente au moins une zone d'extrémité s'inclinant à un angle prédéterminé par rapport à une zone linéaire,
**caractérisé en ce que**
l'au moins une zone d'extrémité du noyau (12a) est fléchie le long d'une configuration d'un boîtier (10) qui contient l'antenne réceptrice (12).

**2.** Endoscope capsulé selon la revendication 1, dans lequel :

une pluralité d'antennes réceptrices (12) sont prévues ; et
les bobines réceptrices (12b) de la pluralité d'antennes réceptrices (12) sont connectées en série ou en parallèle.

3. Endoscope capsulé selon la revendication 2, dans lequel la pluralité d'antennes réceptrices (12) sont agencées le plus loin possible les unes des autres dans le boîtier (10).

4. Système électronique capsulé comprenant :

un système d'alimentation en énergie (2) équipé d'une antenne d'émission (5, 5a, 5b) pour transmettre sans fil une énergie électrique d'une source d'énergie (6) ; et
un endoscope capsulé (3) qui contient une antenne réceptrice (12) ayant une bobine réceptrice (12b) enroulée autour d'une périphérie externe d'un noyau sensiblement en forme de barre (12a) pour recevoir l'énergie électrique transmise, dans lequel le noyau (12a) de l'antenne réceptrice (12) présente au moins une zone d'extrémité s'inclinant à un angle prédéterminé par rapport à une zone linéaire,
**caractérisé en ce que**
l'au moins une zone d'extrémité du noyau (12a) est fléchie le long d'une configuration d'un boîtier (10) qui contient l'antenne réceptrice (12).

5. Système endoscopique capsulé selon la revendication 4, dans lequel :

une pluralité d'antennes électriques (12) sont prévues ; et
les bobines réceptrices (12b) de la pluralité d'antennes réceptrices (12) sont connectées en série ou en parallèle.

6. Système endoscopique capsulé selon la revendication 5, dans lequel la pluralité d'antennes réceptrices (12) sont agencées le plus loin possible les unes des autres dans le boîtier (10).

EP 1 952 754 B1

# FIG.1

# FIG.2

EP 1 952 754 B1

# FIG.3

# FIG.4

13

## FIG.5

12A

1.0mm

12a

15.0mm

5.0mm

12b

0.5mm

## FIG.6

12B

1.0mm

7.5mm

12a

15.0mm

5.0mm

12b

0.5mm

30°

7.5mm

1.0mm

14

*12C*

FIG.7

1.0mm

7.5mm

*12a*

5.0mm

*12b*

15.0mm

0.5mm

45°

7.5mm

1.0mm

*12D*

FIG.8

1.0mm

3.75mm

*12a*

5.0mm

*12b*

15.0mm

0.5mm

3.75mm

30°

1.0mm

# FIG.9

# FIG.10

# FIG.11

# FIG.12

# FIG.13

# FIG.14

# FIG.15

# FIG.16

EP 1 952 754 B1

# FIG.17

# FIG.18

20

# FIG.19

# FIG.20

# FIG.21

## FIG.22

## FIG.23

## FIG.24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001224551 A
- JP 2006149687 A